**Europäisches Patentamt**

**European Patent Office**

**Office européen des brevets**

(19)

(11) Veröffentlichungsnummer: **0 029 191**
**B1**

(12) **EUROPÄISCHE PATENTSCHRIFT**

(45) Veröffentlichungstag der Patentschrift:
25.04.84

(51) Int. Cl.³: **C 07 G 7/00**, A 61 K 37/02, A 61 K 39/00, G 01 N 33/54

(21) Anmeldenummer: 80106897.4

(22) Anmeldetag: 08.11.80

---

(54) Neues Protein PP11, ein Verfahren zu seiner Gewinnung und seine Verwendung.

---

(30) Priorität: 17.11.79 DE 2946458

(43) Veröffentlichungstag der Anmeldung:
27.05.81 Patentblatt 81/21

(45) Bekanntmachung des Hinweises auf die Patenterteilung:
25.04.84 Patentblatt 84/17

(84) Benannte Vertragsstaaten:
AT DE FR GB IT

(56) Entgegenhaltungen:
EP - A - 0 009 715
DE - A - 2 854 759

(73) Patentinhaber: BEHRINGWERKE AKTIENGESELLSCHAFT, Postfach 1140, D-3550 Marburg/Lahn (DE)

(72) Erfinder: Bohn, Hans, Dr., Oberer Eichweg 26, D-3550 Marburg 1 (DE)

(74) Vertreter: Meyer-Dulheuer, Karl-Hermann, Dr. et al, HOECHST Aktiengesellschaft Zentrale Patentabteilung Postfach 80 03 20, D-6230 Frankfurt/Main 80 (DE)

EP 0 029 191 B1

BUNDESDRUCKEREI BERLIN

Neues Protein PP₁₁, ein Verfahren zu seiner Gewinnung und seine Verwendung

Die Erfindung betrifft ein neues Protein (PP₁₁), ein Verfahren zu dessen Herstellung sowie seine Verwendung.

Gegenstand der Erfindung ist das Protein PP₁₁, gekennzeichnet durch

a) einen Kohlenhydratanteil von 3,9±0,9%, bestehend aus 2,6±0,5% Hexosen, 1,0±0,3% Hexosaminen, 0,05±0,03% Fucose und 0,26±0,07% Neuraminsäure;

b) einen Sedimentationskoeffizienten $S^0_{20,w}$ von 3,5±0,2 S;

c) ein in der Ultrazentrifuge bestimmtes Molekulargewicht von 44 300±6000;

d) ein im Natriumdodecylsulfat (SDS)-haltigen Polyacrylamidgel bestimmtes Molekulargewicht von 62 000±3000;

e) einen Extinktionskoeffizienten $E^{1\%}_{1cm}$ (280 nm) von 13,4±1,0 und

f) eine elektrophoretische Beweglichkeit im Bereich der $\alpha_1$-Globuline.

Zur Erläuterung der kennzeichnenden Merkmale des Proteins sei folgendes ausgeführt:

Der Sedimentationskoeffizient wurde in einer analytischen Ultrazentrifuge der Firma Beckman (Spinco-Apparatur, Modell E) bei 60.000 UpM in Doppelsektorzellen mit Hilfe der UV-Scanner Technik bei 280 nm bestimmt. Als Lösungsmittel diente ein 0,05 M Phosphatpuffer (pH 6,8), der 0,2 Mol/l NaCl enthielt. Die Proteinkonzentration wurde auf eine optische Dichte von etwa 3 eingestellt. Der Sedimentationskoeffizient wurde auf die Basis von Wasser bei 20° C umgerechnet.

Zur Ermittlung des Molekulargewichts in der Ultrazentrifuge wurde die Sedimentationsgleichgewichtsmethode herangezogen. Die Konzentration des Proteins ist dabei auf eine optische Dichte von etwa 1,0 eingestellt worden. Die Bestimmung wurde bei 9.000 UpM vorgenommen. Die Registrierung erfolgte mit UV-Optik bei 280 nm unter Einsatz des photoelektrischen Scanners.

Zur Bestimmung des Molekulargewichts im SDS-PAA-Gel wurde ein Gel mit 7,5 g/100 ml Polyacrylamid (PAA), das 0,1 g/100 ml Natriumdodecylsulfat (SDS) enthielt, verwendet. Als Vergleichssubstanz dienten humanes Plazentalactogen (HPL) und Human-Albumin sowie dessen Aggregate.

Zur Bestimmung des Extinktionskoeffizienten wurde 0,1 g Substanz in 100 ml destilliertem Wasser gelöst.

Die elektrophoretische Beweglichkeit wurde in der Mikromodifikation mit dem Gerät Microzone R 200 von Beckman Instruments auf Zelluloseacetatfolien (Fa. Sartorius) unter Verwendung von Natriumdiäthylbarbiturat-Puffer, pH 8,6, bestimmt.

Die Bestimmung der Kohlenhydrate erfolgte nach der von H. E. Schultze, R. Schmidtberger, H. Haupt, Biochem. Z., 329, Seite 490 (1958), beschriebenen Methode.

Die Aminosäurenanalyse wurde nach S. Moore, D. H. Spackman, W. H. Stein, Anal. Chem. 30, Seite 1185 (1958), unter Verwendung des Flüssigkeitschromatographen Multichrom B der Firma Beckman durchgeführt. Cystin wurde nach Oxydation des Proteins mit Perameisensäure [S. Moore et al., Anal. Chem., 238, Seite 235 (1963)] als Cysteinsäure bestimmt. Der Tryptophangehalt ist direkt photometrisch nach H. Edelhoch, Biochemistry, 6, Seite 1948 (1967), ermittelt worden.

Tabelle I enthält das Ergebnis der Aminosäurenanalyse von PP₁₁.

Tabelle I

Aminosäurenzusammensetzung von PP₁₁
(Reste pro 100 Reste in Mol-%)

|  |  | VK% (Variationskoeffizient) |
|---|---|---|
| Lysin | 6,26 | 7,00 |
| Histidin | 3,34 | 1,72 |
| Arginin | 3,31 | 5,60 |
| Asparaginsäure | 10,75 | 2,43 |
| Threonin | 3,31 | 10,49 |
| Serin | 9,63 | 1,84 |
| Glutaminsäure | 13,81 | 2,09 |
| Prolin | 4,10 | 4,68 |
| Glycin | 6,23 | 6,26 |
| Alanin | 6,30 | 1,82 |
| Cystin $^1/_2$ | 3,37 | 4,53 |
| Valin | 4,53 | 5,40 |
| Methionin | 1,00 | 26,22 |
| Isoleucin | 3,60 | 2,24 |
| Leucin | 6,74 | 1,05 |
| Tyrosin | 5,90 | 3,02 |
| Phenylalanin | 6,06 | 2,52 |
| Tryptophan | 1,66 | 20,87 |

$PP_{11}$ hat folgende Eigenschaften, die sich zu seiner Isolierung verwenden lassen.

1) Mit Ammoniumsulfat wird es bei pH 7,0 und 30–60% Sättigung aus wäßrigen Lösungen gefällt.
2) Mit wasserlöslichen Acridinbasen, z. B. 2-Äthoxi-6,9-diaminoacridinlactat (Rivanol®) wird es bei pH-Werten zwischen 4–9 und einer Konzentration der Base von 0,2 bis 0,8 g/100 ml präzipitiert.
3) Unter den Bedingungen einer Euglobulinfällung, nämlich durch Einstellen eines pH-Wertes von 5–6, in einer verdünnten Pufferlösung wird es nicht präzipitiert.
4) In der präparativen Elektrophorese wandert es im Bereich der $\alpha_1$-Globuline.
5) Bei der Gelfiltration mit Sephadex® verhält es sich wie Proteine mit Molekulargewichten von 30 000 bis 90 000.
6) Es läßt sich an schwach basische Ionenaustauscher wie beispielsweise DEAE-Cellulose oder DEAE-Sephadex bei einer Leitfähigkeit von etwa 0–2 mS und einem pH-Wert von etwa pH 7 bis 9 binden.
7) Es kann aus einer wäßrigen Lösung durch Immunadsorption angereichert und isoliert werden.

Gegenstand der Erfindung ist ferner ein Verfahren zur Gewinnung oder Anreicherung des $PP_{11}$, dadurch gekennzeichnet, daß ein aus menschlichen Plazenten mittels einer Kochsalzlösung erhaltener Extrakt, einer oder mehreren der folgenden Maßnahmen unterworfen wird:

a) Fällung des Proteins $PP_{11}$ mit Ammoniumsulfat im pH-Bereich von 5 bis 8 und bei 30–60% Sättigung;
b) Fällung des Proteins $PP_{11}$ mit einer wasserlöslichen Acridinbase bei einem pH-Wert zwischen 4 und 9 und einer Konzentration der Base von 0,2–0,8 g/100 ml;
c) Euglobulinfällung bei einem pH-Wert von 5–6 in einer verdünnten Salzlösung, wobei Begleitproteine abgetrennt werden und $PP_{11}$ nicht präzipiert wird;
d) präparative Zonenelektrophorese, wobei die »$\alpha_1$-Globulinfraktion« gewonnen wird;
e) Gelfiltration oder Ultrafiltration, wobei Proteine im Molekulargewichtsbereich von 30 000 bis 90 000 gewonnen werden;
f) Adsorption an einem schwach basischen Ionenaustauscher und Elution des Proteins $PP_{11}$;
g) immunadsorptive Anreicherung.

Neben Ammoniumsulfat können auch andere in der präparativen Biochemie üblicherweise eingesetzten Neutralsalze zur Ausfällung des $PP_{11}$ verwendet werden. Neben einer Acridinbase ist auch ein wasserlösliches Derivat einer Chinolinbase, wie sie für Proteinfraktionierungen bekannt sind, einsetzbar. Entsprechend seinem elektrophoretischen Verhalten wie seinem Molekulargewicht sind zur Isolierung des Proteins auch andere Maßnahmen brauchbar, die geeignet sind, ein $\alpha_1$-Globulin von anderen Proteinen abzutrennen. Hierzu können die verschiedenen Methoden der Gelfiltration oder Ultrafiltration oder auch die Eigenschaft des $PP_{11}$ an schwach basische Ionenaustauscher gebunden und hiervon wieder eluiert werden zu können, verwendet werden.

Durch eine zweckmäßige Kombination der genannten Maßnahmen, die eine Anreicherung des $PP_{11}$ oder eine Abtrennung dieses Proteins von anderen Proteinen bewirken, kann das $PP_{11}$ isoliert werden.

Zum Nachweis und zur Bestimmung des $PP_{11}$ etwa in einer Fraktion aus einer Trennoperation können neben den angegebenen Parametern auch immunchemische Methoden dienen, da $PP_{11}$ antigene Eigenschaften hat.

Ein für diesen Zweck brauchbares Antiserum kann folgendermaßen gewonnen werden: Durch Immunisieren von Kaninchen mit einer $PP_{11}$-enthaltenden Plazentaproteinfraktion (Mutterlaugen aus der Kristallisation von humanem Plazentalactogen (HPL) nach Bohn, H., Experientia 27 (1971), 1223) wird ein polyvalentes Antiserum erhalten, mit dem $PP_{11}$ nachgewiesen werden kann. Dieses Antiserum kann durch Absorption mit normalem menschlichen Serum und solchen Plazentafraktionen, die $PP_{11}$ nicht enthalten, oder Proteinen, beispielsweise mit HPL, gegen das Antigen $PP_{11}$ weitgehend spezifisch gemacht werden. Dieses spezifische Antiserum kann einerseits zum immunologischen Nachweis des $PP_{11}$, andererseits zur Herstellung eines Immunadsorbens dienen, das zur Anreicherung und Isolierung von $PP_{11}$ eingesetzt werden kann.

Zum immunologischen Nachweis von $PP_{11}$ kann die Geldiffusionstechnik nach Ouchterlony (vgl. Schultze and Heremans, Molecular Biology of Human Proteins, Vol. 1, pg. 134) herangezogen werden.

Mit Hilfe des nach der vorliegenden Anmeldung gewonnenen $PP_{11}$ lassen sich durch Immunisieren von Tieren nach bekannten Methoden monospezifische Antiseren herstellen.

$PP_{11}$ hat antigene Eigenschaften. Bei der Immunisierung von Tieren mit diesem Protein werden spezifische Antikörper gebildet. Der Nachweis und die Bestimmung von $PP_{11}$ mit immunologischen Methoden hat diagnostische Bedeutung: einerseits zur Überwachung von Schwangerschaften, andererseits zum Nachweis, speziell von trophoblastischen, aber auch von nichttrophoblastischen Tumoren sowie zur Überwachung des Krankheitsverlaufs und zur Kontrolle der Therapie bei solchen Erkrankungen.

$PP_{11}$ kann also verwendet werden, um Antiseren herzustellen, die dazu dienen können, $PP_{11}$ nachzuweisen und zu bestimmen.

Die Erfindung wird am nachstehenden Beispiel erläutert:

## Beispiel

### A) Extraktion der Plazenten und Fraktionierung des Extraktes mit Rivanol und Ammoniumsulfat

1000 kg tiefgefrorene menschliche Plazenten werden im Schneidmischer zerkleinert und mit 1000 l einer Kochsalzlösung (4 g/kg) extrahiert. Der Extrakt wird, nach Abtrennung des Gewerberückstandes durch Zentrifugation, mit Essigsäure (200 g/kg) auf pH 6,0 eingestellt und unter Rühren mit 200 l einer Lösung (30 g/kg) von 2-Äthoxy-6,9-Diaminoacridin-Lactat (Rivanol®, Hoechst AG) versetzt. Den durch Zentrifugation abgetrennten Niederschlag versetzt man mit 500 l einer NaCl-Lösung (25 g/kg), rührt 4 Stunden und zentrifugiert vom abgeschiedenen Chlorid des 2-Äthoxy-6,9-Diaminoacridins ab. Die Lösung versetzt man unter Rühren langsam mit soviel festem Ammoniumsulfat, daß eine Endkonzentration von 300 g/l erreicht wird, wodurch $PP_{11}$ zusammen mit anderen Proteinen ausfällt. Der Niederschlag wird abzentrifugiert. Man erhält etwa 4,5 kg einer feuchten Paste, die im Nachfolgenden als Fraktion A bezeichnet wird.

### B) Gelfiltration an Sephadex G-150

1500 g der Fraktion A werden in Wasser gelöst und gegen einen 0,01 M Tris-HC1-Puffer (pH 8,0), der 0,5 g/l $NaN_3$ enthält (Pufferlösung I) dialysiert. Die zurückbleibende Lösung wird auf eine mit Sephadex® G-150 gefüllte Säule (60 × 56 cm) aufgetragen und mit Pufferlösung I eluiert. Die Eluate werden im Geldiffusionstest nach Ouchterlony mit einem spezifischen Anti-$PP_{11}$-Kaninchenserum geprüft. Die $PP_{11}$ enthaltenden Fraktionen werden gesammelt und als Fraktion B bezeichnet.

### C) Chromatographie an DEAE-Cellulose

Fraktion B wird an DEAE-Cellulose (Säule 10 × 28 cm) adsorbiert. Man spült die Säule mit Pufferlösung I und eluiert mit Kochsalzlösung (8,5 g/l) solange, bis im Durchlauf mit Trichloressigsäure keine Fällung mehr entsteht. Aus dem Eluat werden die Proteine durch Zusatz von soviel Ammoniumsulfat, daß die Konzentration 300 g/l beträgt, ausgefällt. Der Niederschlag wird abzentrifugiert (Fraktion C).

### D) Euglobulinfällung

Fraktion C wird in Wasser gelöst und gegen Pufferlösung I dialysiert. Man stellt die Lösung durch Zugabe von 2 N Essigsäure unter Rühren auf pH 5,5 ein. Der Niederschlag, der im wesentlichen nur Begleitproteine enthält, wird abzentrifugiert. Der Überstand wird gegen eine 0,1 M Tris-HC1-Pufferlösung von pH 8, die 1 Mol/l NaCl und 1 g/l Natriumacid enthält, (Pufferlösung II) dialysiert (Fraktion D).

### E) Anreicherung von $PP_{11}$ durch Immunadsorption

#### 1. Herstellung des Immunadsorbens

450 ml eines Anti-$PP_{11}$-Serums vom Kaninchen werden gegen 0,02 M Phosphatpuffer (pH 7,0) dialysiert und zur Abtrennung der Immunglobuline an DEAE-Cellulose chromatographiert. Die Immunglobulinfraktion (6,28 g Protein) wird dann mit 628 g besonders gereinigter Agarose in Kugelform (Sepharose® der Pharmacia, Uppsala, Schweden), die mit 78,5 g Bromcyan aktiviert worden war, umgesetzt und so kovalent an einen Träger gebunden. Das Verfahren wurde beschrieben von Axen, R., Porath, J., Ernbach S., Nature 214, 1302 (1967). Mit Hilfe eines auf diese Weise hergestellten Immunadsorbens kann $PP_{11}$ aus seinen Lösungen, insbesondere aus $PP_{11}$ angereicherten Plazentaextraktfraktionen isoliert werden.

#### 2. Immunadsorption

Das Immunadsorbens wird in Pufferlösung II suspendiert, in eine Chromatographiesäule gefüllt (6 × 20 cm) und mit Pufferlösung II nachgespült. Dann läßt man langsam 40 ml Fraktion B durch die Säule wandern, wobei $PP_{11}$ immunadsorptiv gebunden wird. Man wäscht die Säule gründlich mit Puffer II nach und eluiert das adsorbierte Protein mit etwa 600 ml 3 mol/l Kaliumrhodanid-Lösung. Die $PP_{11}$-haltigen Eluate werden gegen Pufferlösung II dialysiert und mit einem Ultrafilter auf etwa 10 ml eingeengt. Ausbeute pro Adsorption ~25 mg $PP_{11}$.

Das Adsorbens in der Säule wird unmittelbar nach der Elution von $PP_{11}$ wieder mit Pufferlösung II neutralisiert und gründlich gewaschen; es kann dann erneut zur immunadsorptiven Bindung von $PP_{11}$ eingesetzt werden.

### F) Hochreinigung von $PP_{11}$

Das durch Immunadsorption gewonnene Protein ist häufig durch unspezifisch gebundene Serumproteine und andere Plazenta-Gewebsproteine verunreinigt. Die Abtrennung der Hauptmenge der Serum-Begleitproteine gelingt beispielsweise durch Gelfiltration an Sephadex®G-150. Die restlichen Begleitproteine werden dann durch inverse oder negative Immunadsorption, d. h. mit Hilfe von trägergebundenen Antikörpern gegen die noch als Verunreinigung vorliegenden Proteine, Immunglobuline (IgG), $\beta_2$-Glykoprotein III, $PP_{10}$ und $PP_{12}$, entfernt.

**Patentansprüche für die Vertragsstaaten: DE, FR, GB, IT**

1. Protein PP$_{11}$ gekennzeichnet durch

a)  einen Kohlenhydratanteil von 3,9±0,9% bestehend aus 2,6±0,5% Hexosen, 1,0±0,3% Hexosaminen, 0,05±0,03% Fucose und 0,26±0,07% Neuraminsäure,

b)  einen Sedimentationskoeffizienten S$^{0}_{20,w}$ von 3,5±0,2 S,

c)  ein in der Ultrazentrifuge bestimmtes Molekulargewicht von 44 300±6000,

d)  ein im Natriumdodecylsulfat (SDS)-haltigen Polyacrylamidgel bestimmtes Molekulargewicht von 62 000±3000,

e)  einen Extinktionskoeffizienten E$^{1\%}_{1cm}$ (280 nm) 13,4±1,0 und

f)  eine elektrophoretische Beweglichkeit im Bereich der $\alpha_1$-Globuline.

2. Verfahren zur Gewinnung oder Anreicherung des Proteins PP$_{11}$ nach Anspruch 1, dadurch gekennzeichnet, daß ein aus menschlichen Plazenten mittels einer Kochsalzlösung erhaltener Extrakt einer oder mehreren der folgenden Maßnahmen unterworfen wird:

a)  Fällung des Proteins PP$_{11}$ mit Ammoniumsulfat im pH-Bereich von 5 bis 8 und bei 30−60% Sättigung;

b)  Fällung des Proteins PP$_{11}$ mit einer wasserlöslichen Acridinbase bei einem pH-Wert zwischen 4 und 9 und einer Konzentration der Base von 0,2−0,8 g/100 ml;

c)  Euglobulinfällung bei einem pH-Wert von 5−6 in einer verdünnten Salzlösung, wobei Begleitproteine abgetrennt werden und PP$_{11}$ nicht präzipiert wird;

d)  präparative Zonenelektrophorese, wobei die »$\alpha_1$-Globulinfraktion« gewonnen wird;

e)  Gelfiltration oder Ultrafiltration, wobei Proteine im Molekulargewichtsbereich von 30 000 bis 90 000 gewonnen werden;

f)  Adsorption an einem schwach basischen Ionenaustauscher und Elution des Proteins PP$_{11}$;

g)  immunadsorptive Anreicherung.

3. Verwendung des Proteins nach Anspruch 1 zur Gewinnung eines Antiserums zum Nachweis und zur Bestimmung dieses Proteins.

**Patentansprüche für den Vertragsstaat: AT**

1. Verfahren zur Gewinnung oder Anreicherung des Proteins PP$_{11}$, das

a)  einen Kohlenhydratanteil von 3,9±0,9% bestehend aus 2,6±0,5% Hexosen, 1,0±0,3% Hexosaminen, 0,05±0,03% Fucose und 0,26±0,07% Neuraminsäure,

b)  einen Sedimentationskoeffizienten S$^{0}_{20,w}$ von 3,5±0,2 S,

c)  ein in der Ultrazentrifuge bestimmtes Molekulargewicht von 44 300±6000,

d)  ein im Natriumdodecylsulfat (SDS)-haltigen Polyacrylamidgel bestimmtes Molekulargewicht von 62 000±3000,

e)  einen Extinktionskoeffizienten E$^{1\%}_{1cm}$ (280 nm) 13,4±1,0 und

f)  eine elektrophoretische Beweglichkeit im Bereich der $\alpha_1$-Globuline hat,

dadurch gekennzeichnet, daß ein aus menschlichen Plazenten mittels einer Kochsalzlösung erhaltener Extrakt einer oder mehreren der folgenden Maßnahmen unterworfen wird:

a)  Fällung des Proteins PP$_{11}$ mit Ammoniumsulfat im pH-Bereich von 5 bis 8 und bei 30−60% Sättigung;

b)  Fällung des Proteins PP$_{11}$ mit einer wasserlöslichen Acridinbase bei einem pH-Wert zwischen 4 und 9 und einer Konzentration der Base von 0,2−0,8 g/100 ml;

c)  Euglobulinfällung bei einem pH-Wert von 5−6 in einer verdünnten Salzlösung, wobei Begleitproteine abgetrennt werden und PP$_{11}$ nicht präzipiert wird;

d)  präparative Zonenelektrophorese, wobei die »$\alpha_1$-Globulinfraktion« gewonnen wird;

e)  Gelfiltration oder Ultrafiltration, wobei Proteine im Molekulargewichtsbereich von 30 000 bis 90 000 gewonnen werden;

f)  Adsorption an einem schwach basischen Ionenaustauscher und Elution des Proteins PP$_{11}$;

g)  immunadsorptive Anreicherung.

2. Verwendung des Proteins nach Anspruch 1 zur Gewinnung eines Antiserums zum Nachweis und zur Bestimmung dieses Proteins.

**Claims for the Constracting States DE, FR, GB, IT**

1. Protein PP$_{11}$ characterized by

a)  a content of carbohydrates of 3,9±0,9% of which are 2,6±0,5% hexoses, 1,0±0,3% hexosamines 0,05±0,03% fucose and 0,26±0,07% neuraminic acid,

b)  a sedimentation coefficient S$^{0}_{20,w}$ of 3,5±0,2 S,

c)  a molecular weight of 44 300±6000 determined in an ultracentrifuge,

d)  a molecular weight of 62 000±3000 determined in polyacrylamide gel containing sodium dodecylsulfate (SDS),

e)  an extinktion coefficient E$^{1\%}_{1cm}$ (280 nm) of 13,4±1,0 and

f)  an electrophoretic mobility in the range of the $\alpha_1$-globulins.

2. Process for preparing or enriching the protein PP$_{11}$ according to claim 1, which comprises subjecting an extract obtained from human placenta by means of a saline solution to one or more of the following measures:

a) precipitating the Protein $PP_{11}$ with ammonium sulfate in the pH-range of from 5 to 8 and at a saturation of 30 to 60%;

b) precipitating the Protein $PP_{11}$ with a water-soluble acridine base at a pH-value of between 4 and 9 and a concentration of the base of from 0.2 to 0.8 g/100 ml;

c) euglobulin precipitation at a pH of from 5 to 6 in a diluted salt solution, whereby accompanying proteins are separated and $PP_9$ is not precipitated;

d) preparative zone electrophoresis, whereby the »$\beta_1$-globulin fraction« is isolated;

e) gel-filtration or ultrafiltration, whereby proteins in the molecular weight range of from 30 000 to 90 000 are isolated;

f) adsorbing $PP_{11}$ on a weakly basic ion exchanger and eluting it;

g) immuno-adsorptive enrichment of $PP_{11}$.

3. Use of the protein according to claim 1 for preparing an antiserum for detecting and determining this protein.

## Claims for the Contracting State Austria

1. Process for preparing or enriching the Protein $PP_{11}$ characterized by

a) a content of carbohydrates of $3,9 \pm 0,9\%$ of which are $2,6 \pm 0,5\%$ hexoses, $1,0 \pm 0,3\%$ hexosamines, $0,05 \pm 0,03\%$ fucose and $0,26 \pm 0,07\%$ neuraminic acid,

b) a sedimentation coefficient $S^o_{21,w}$ of $3,5 \pm 0,2$ S,

c) a molecular weight of $44\ 300 \pm 6000$ determined in an ultracentrifuge,

d) a molecular weight of $62\ 000 \pm 3000$ determined in polyacrylamide gel containing sodium dodecylsulfate (SDS),

e) an extinktion coefficient $E^{1\%}_{1cm}$ (280 nm) of $13,4 \pm 1,0$ and

f) an electrophoretic mobility in the range of the $\alpha_1$-globulins,

which comprises subjecting an extract obtained from human placenta by means of a saline solution to one or more of the following measures:

a) precipitating the Protein $PP_{11}$ with ammonium sulfate in the pH-range of from 5 to 8 and at a saturation of 30 to 60%;

b) precipitating the Protein $PP_{11}$ with a water-soluble acridine base at a pH-value of between 4 and 9 and a concentration of the base of from 0.2 to 0.8 g/100 ml;

c) euglobulin precipitation at a pH of from 5 to 6 in a diluted salt solution, whereby accompanying proteins are separated and $PP_9$ is not precipitated;

d) preparative zone electrophoresis, whereby

the »$\beta_1$-globulin fraction« is isolated;

e) gel-filtration or ultrafiltration, whereby proteins in the molecular weight range of from 30 000 to 90 000 are isolated;

f) adsorbing $PP_{11}$ on a weakly basic ion exchanger and eluting it;

g) immuno-adsorptive enrichment of $PP_{11}$.

2. Use of the protein according to claim 1 for preparing an antiserum for detecting and determining this protein.

## Revendications pour les Etats Contractants: DE, FR, GB, IT

1. Protéine $PP_{11}$, caractérisée par:

a) une teneur en hydrates de carbone de $3,9 \pm 0,9\%$ consistant en $2,6 \pm 0,5\%$ d'hexosamines, $0,05 \pm 0,03\%$ de fucose et $0,26 \pm 0,07\%$ d'acide neuraminique,

b) un coefficient de sédimentation $S^o_{20,w}$ de $3,5 \pm 0,2$ S,

c) une masse moléculaire déterminée par ultracentrifugation de $44\ 300 \pm 6000$,

d) une masse moléculaire déterminée dans un gel de polyacrylamide contenant du dodécyl sulfate de sodium (SDS) de $62\ 000 \pm 3000$,

e) un coefficient d'extinction $E^{1\%}_{1cm}$ (280 nm) de $13,4 \pm 1,0$ et

f) une mobilité électrophorétique dans le domaine des $\alpha_1$-globulines.

2. Procédé pour l'isolement ou l'enrichissement de la protéine $PP_{11}$ selon la revendication 1, caractérisé en ce qu'on soument un extrait obtenu à partir de placentas humains traités par une solution saline, à une ou plusieurs des opérations suivantes:

a) précipitation de la protéine $PP_{11}$ avec du sulfate d'ammonium dans une gamme de pH de 5 à 8 et à une saturation de 30 à 60%;

b) précipitation de la protéine $PP_{11}$ avec une base d'acridine soluble dans l'eau à un pH compris entre 4 et 9 et à une concentration de la base de 0,2 à 0,8 g/100 ml;

c) précipitation des euglobulines à un pH de 5 à 6 dans une solution saline diluée, les protéines secondaires étant alors séparées et $PP_{11}$ n'étant pas précipitée;

d) électrophorèse de zone de préparation pour obtenir la fraction »$\alpha_1$-globuline«;

e) filtration sur gel ou ultrafiltration, pour obtenir des protéines ayant une masse moléculaire comprise entre 30 000 et 90 000;

f) adsorption sur un échangeur d'ion faiblement basique et élution de la protéine $PP_{11}$;

g) enrichissement par immunoadsorption.

3. Utilisation de la protéine selon la revendication 1, pour la production d'un antisérum pour la détection et le dosage de cette protéine.

**Revendications pour l'Etat Contractant AT**

1. Procédé pour l'isolement ou l'enrichissement de la protéine PP$_{11}$ qui a:

a) une teneur en hydrates de carbone de 3,9±0,9%, consistant en 2,6±0,5% d'hexoses, 1,0±0,3% d'hexosamines, 0,05±0,03% de fucose et 0,26±0,07% d'acide neuraminique,

b) un coefficient de sédimentation $S^0_{20,w}$ de 3,5±0,2 S,

c) une masse moléculaire déterminée par ultracentrifugation de 44 300±6000,

d) une masse moléculaire déterminée dans un gel de polyacrylamide contenant du dodécyl sulfate de sodium (SDS) de 62 000±3000,

e) un coefficient d'extinction $E^{1\%}_{1cm}$ (280 nm) de 13,4±1,0 et

f) une mobilité électrophorétique dans le domaine des $\alpha_1$-globulines,

caractérisé en ce qu'on soumet un extrait obtenu à partir de placentas humains traités avec une solution saline, à l'une ou à plusieurs des opérations suivantes:

a) précipitation de la protéine PP$_{11}$ avec du sulfate d'ammonium dans une gamme de pH de 5 à 8 et à une saturation de 30 à 60%;

b) précipitation de la protéine PP$_{11}$ avec une base d'acridine soluble dans l'eau à un pH compris entre 4 et 9 et à une concentration de la base de 0,2 à 0,8 g/100 ml;

c) précipitation des euglobulines à un pH de 5 à 6 dans une solution saline diluée, les protéines secondaires étant alors séparées et la PP$_{11}$ n'étant pas précipitée;

d) électrophorèse de zone de préparation pour obtenir la fraction »$\alpha_1$-globuline«;

e) filtration sur gel ou ultrafiltration pour obtenir des protéines ayant une masse moléculaire comprise entre 30 000 et 90 000;

f) adsorption sur un échangeur d'ion faiblement basique et élution de la protéine PP$_{11}$;

g) enrichissement par immunoadsorption.

2. Utilisation de la protéine selon la revendication 1 pour la production d'un antisérum pour la détection et le dosage de cette protéine.